# FASCICULE DE BREVET EUROPEEN

(11) **EP 0 704 205 B1**
(45) Date de publication et mention de la délivrance du brevet: **19.03.1997**
(21) Numéro de dépôt: 95401959.2
(22) Date de dépôt: 25.08.1995
(51) Int. Cl.: A61K 7/48

(54) **Composition cosmétique anhydre résistante à l'eau**
Wasserbeständige wasserfreie kosmetische Zusammensetzung
Water resistant anhydrous cosmetic composition

(30) Priorité: 30.09.1994 FR 9411743
(43) Date de publication de la demande: 03.04.1996
(73) Titulaire: L'OREAL, 75008 Paris (FR)
(72) Inventeur: Mellul, Myriam, F-92240 L'Hay Les Roses (FR); Thau, Paul, Berkeley Heights, New Jersey 07922 (US); Fehn, Paul, Westfield, New Jersey 07090 (US); Pinzon, Carlos, Hackensack, New Jersey 07601 (US)
(74) Mandataire: Dodin, Catherine

(56) Documents cités:
- EP-A- 0 133 963
- EP-A- 0 521 647
- EP-A- 0 548 694
- FR-A- 2 688 134

## Description

L'invention concerne des compositions de maquillage anhydres et résistantes à l'eau comprenant au moins une gomme de silicone.

On connaît dans l'art antérieur, des compositions résistantes à l'eau, sous forme d'émulsion eau-dans-huile ou eau-dans-silicone. L'utilisation de telles émulsions s'appuie sur le fait qu'après étalement, l'eau s'évapore et l'huile ou la silicone reste au contact de la peau, procurant un maquillage résistant à l'eau.
L'application de telles compositions présente toutefois l'inconvénient de laisser à la peau un aspect huileux et de procurer au toucher un effet gras. Le résultat obtenu après application n'est pas réellement naturel, le maquillage brille ou devient brillant.
Une autre forme de compositions cosmétiques résistantes à l'eau est une composition dans laquelle des charges, dont des pigments, sont introduites, dans une huile de silicone volatile ou non. L'inconvénient majeur de ce type de composition réside dans l'effet de tiraillement de la peau, et de sensation de sec peu agréable, qui apparaît après application. De plus, souvent, l'effet recherché dans l'utilisation de telles compositions contenant des pigments, à savoir une coloration de la peau, n'est pas atteint. La nature même de la composition fait que la répartition des pigments dans la composition après étalement n'est pas homogène; les pigments s'étalent plus ou moins uniformément et ont tendance à s'agglomérer dans les pores et les plis cutanés. Cet effet va à l'encontre de la recherche d'une coloration proche de celle obtenue naturellement.
Dans tous les cas, les compositions obtenues présentent des défauts tant au niveau de la stabilité qu'au niveau de l'homogénéité de la dispersion des pigments. Des solutions à ces problèmes ont été proposées, comme par exemple l'utilisation de colorants hydrosolubles ou de dihydroxyacétone (DHA). Dans le cas des colorants hydrosolubles les maquillages obtenus ne sont pas résistants à l'eau, et présentent une homogénéité imparfaite.
Les produits utilisant de la DHA sont difficiles à formuler, du fait de la dégradation et de l'incompatibilité de la DHA avec de nombreux composants habituellement utilisés dans ce genre de compositions. En outre, un inconvénient majeur de la DHA, dans son utilisation dans des compositions cosmétique réside dans l'apparition d'odeurs désagréables au cours du vieillissement.
On connaît également, par le document FR 2688134, une composition cosmétique sous forme de poudre, comprenant des huiles de silicone, des cires de silicone, des résines de silicone, et éventuellement des gommes de silicone.
La présente invention a pour but d'apporter des solutions aux différents problèmes rencontrés dans les applications de l'art antérieur et de proposer une composition cosmétique possédant une bonne résistance à l'eau tout en possédant de bonnes propriétés cosmétiques.
De manière surprenante et inattendue, la demanderesse a pu montrer qu'il est possible d'obtenir des compositions de maquillage anhydres, résistantes à l'eau, présentant une grande homogénéité en particulier à l'étalement, en mélangeant au moins une grande proportion de gomme de silicone, une huile de silicone et des pigments.
Plus particulièrement, l'invention a pour objet une composition cosmétique anhydre résistante à l'eau, comprenant 2 à 50% en poids de gomme de silicone de formule ci-après, 10 à 90% en poids d'huile de silicone, 0,5 à 15% en poids de pigments et 0-30% en poids de charges.
Les compositions selon l'invention présentent l'avantage d'être stables dans le temps. Elles sont résistantes à l'eau car anhydres, et ne contiennent ni solvant, ni colorants hydrosolubles. Elles possèdent également une bonne tenue et ne provoquent pas d'effet de tiraillement sur la peau.
On a de plus constaté que de manière surprenante, les pigments présents dans les compositions selon l'invention y étaient dispersés d'une manière très homogène inattendue. Ceci présente donc l'avantage supplémentaire de procurer un maquillage très uniforme et très homogène de la peau.

Dans la suite du texte, les pourcentages seront toujours donnés en poids de matière active par rapport au poids total de la composition.
Dans la composition selon l'invention, la gomme de silicone est présente dans une proportion comprise entre 2 % et 50 % en poids, de préférence entre 4 et 15% et plus particulièrement entre 6 % et 9 %.
La gomme de silicone répond à la formule : 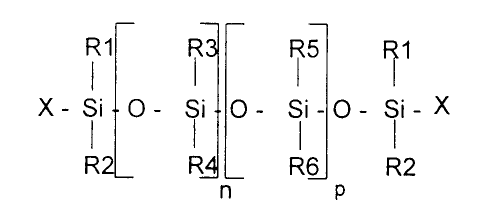 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant 1 à 6 atomes de carbone, ou un radical aryle,
X est un radical alkyle ayant 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.
De manière générale, n et p peuvent prendre, chacun, des valeurs allant de 0 à 5000, de préférence de 0 à 3000.

Parmi les gommes de silicone utilisables selon l'invention, on peut citer celles pour lesquelles :
. les substituants R1 à R6 et X représentent un groupement méthyle, p = 0 et n = 2700, comme celle vendue sous la dénomination SE30 par la société General Electric,
. les substituants R1 à R6 et X représentent un groupement méthyle, p = 0 et n = 2300, comme celle vendue sous la dénomination AK 500000 par la société Waker,
. les substituants R1 à R6 représentent un groupement méthyle, le substituant X représente un groupement hydroxyle, p = 0 et n = 2700, en solution à 13 % dans du cyclopentasiloxane, comme celle vendue sous la dénomination Q2-1401 par la société Dow Corning,
. les substituants R1 à R6 représentent un groupement méthyle, le substituant X représente un groupement hydroxyle, p = 0 et n = 2700, en solution à 13% dans du polydiméthylsiloxane, comme celle vendue sous la dénomination Q2-1403 par la société Dow Corning,
. les substituants R1, R2, R5, R6 et X représentent un groupement méthyle, et les substituants R3 et R4 représentent un groupement aryle tel que le poids moléculaire de la gomme est de l'ordre de 600 000, comme celle vendue sous la dénomination 761 par la société Rhône-Poulenc.

La composition selon l'invention comprend également au moins une huile de silicone, en une proportion de 10 à 90% en poids.
On peut utiliser une huile de silicone volatile ou non volatile.
On peut citer, par exemple :
. des cyclométhicones telles que les cyclométhicones D4, D5 ou D6,
. des polydiméthylsiloxanes (PDMS) de viscosité inférieure à 100 mPa.s et de préférence inférieure à 10 mPa.s,
. des alkyldiméthicones, notamment répondant à la formule: 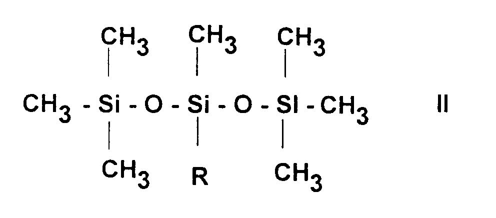 dans laquelle R représente le radical CₙH₂ₙ₊₁, avec n compris entre 3 à 8, et parmi lesquelles on peut citer le X2-1731 de Dow Corning
De préférence, l'huile de silicone est présente dans la composition à raison de 30-70% en poids.
Selon un mode préféré de réalisation de l'invention, on utilise la gomme et l'huile de silicone sous forme d'un prémélange homogène constitué de la gomme de silicone solubilisée dans l'huile de silicone.

La composition selon l'invention comprend également des pigments, en une proportion de 0,5 à 15% en poids, de préférence 2 à 8%.

Les pigments peuvent être blancs ou colorés, minéraux ou organiques.
Les pigments utilisés dans la composition selon l'invention peuvent être enrobés ou non enrobés.
Dans le brevet US 4578566, il est décrit un prétraitement des pigments pour les rendre hydrophobes, comme solution afin d'en introduire de fortes proportions dans des compositions à base de silicone, et afin d'en assurer une meilleure dispersion dans la composition. Ce prétraitement s'effectue en enrobant les pigments avec un polysiloxane. Bien évidement, ce prétraitement augmente non seulement le temps de préparation des compositions mais également leur coût.
Ainsi que cela a été mentionné précédemment, un avantage particulier de l'invention réside dans le fait que les compositions selon l'invention permettent l'obtention d'une dispersion homogène et stable, même lorsque l'on utilise des pigments non enrobés préalablement.
Parmi les pigments utilisables, on peut citer, sans effets limitatifs, le dioxyde de titane (TiO₂), l'oxyde de zinc (ZnO), le dioxyde de zirconium (ZrO₂), les oxydes de fer noir, jaune, rouge, brun, le dioxyde de cérium (CeO₂), ou encore les pigments organiques dits laques de baryum, strontium, calcium, aluminium, et leurs mélanges.

La composition selon l'invention peut comprendre 0-30% de charges. Ces charges peuvent être minérales ou de synthèse, lamellaires ou sphériques.
On peut citer le talc, le mica, la silice, le kaolin, les poudres de Nylon et de polyéthylène, le Téflon, l'amidon, le micatitane, la nacre naturelle, le nitrure de bore, les microsphères creuses telles que l'Expancel de Nobel Industrie.

La composition selon l'invention peut également comprendre des constituants habituellement utilisés dans le genre de compositions cosmétiques envisagé.
Ces constituants sont de préférence choisis en fonction de l'effet cosmétique souhaité pour la composition finale, tel que la couvrance, la transparence, la matité et/ou l'aspect satiné.
On peut citer, sans effets limitatifs :
. les gélifiants comme les argiles modifiées connues sous les noms de bentone, vendues par la société NL Industrie et utilisées telles qu'elles ou préalablement conditionnées dans un gel; la silice hydrophobe; les cires, par exemple de polyéthylène; les sels gras d'aluminium;
. les vitamines comme les tocophérols et leurs dérivés, la vitamine A et ses dérivés, la vitamines C et ses dérivés comme les esters gras dont le palmitate;
. les filtres solaires comme l'octylméthoxycinnamate (Parsol MCX), la 3-benzophénone (Uvinul M40), le butylméthoxydibenzoyl-méthane (Parsol 1789);
. les matériaux huileux comme les huiles végétales, les esters de synthèse, la lécithine, les parfums, les huiles essentielles.
Bien entendu l'homme du métier veillera à choisir ce ou ces éventuels constituants complémentaires, et/ou leur quantité, de telle manière que les propriétés avantageuses de la composition selon l'invention ne soient pas, ou substantiellement pas, altérées par l'adjonction envisagée.

Les procédés de fabrication des compositions selon l'invention ne différent en rien des procédés classiquement utilisés en cosmétique et parfaitement connus de l'homme de l'art.
Les compositions selon l'invention peuvent se présenter sous forme d'un produit de maquillage de la peau tel qu'un fond de teint, un fard à joues, un fard à paupières, un rouge à lèvres, voire sous forme de produit capillaire tel qu'un gel maquillant coiffant.

On va maintenant donner à titre d'exemple des exemples de compositions selon l'invention.

### Exemple 1

On prépare des gels de maquillage ayant les compositions suivantes (en g):

| | Composition A | Composition B | Composition C |
|---|---|---|---|
| A) polydiméthylsiloxane à 12-14 % dans le cyclopentasiloxane (Q2-1401 de Dow) | 67,50 | 67,50 | 67,50 |
| B) TiO₂ + oxydes de fer (non enrobés) | 3,36 | 3,36 | 3,36 |
| C) cyclopentadiméthylsiloxane | 9,50 | 9,50 | -- |
| D) gélifiant | | | |
| . bentone gel IMP (NL Industrie) | 18,64 | 19,14 | -- |
| . bentone gel VS-5 PC (Stéarinerie Dubois) | -- | -- | 28,14 |
| E) charges | | | |
| . microsphères Expancel | 1,00 | 1,00 | 1,00 |
| . Téflon Ceri-dust | -- | 2,00 | -- |

On mélange A et B à la spatule et on passe 3 fois au broyeur à cylindre. On ajoute ensuite C, D et E sous agitation modérée.
On obtient trois formules qui contiennent une proportion importante de composés siliconés et qui permettent d'obtenir, après étalement sur la peau un maquillage coloré et résistant à l'eau.
Bien que les pigments utilisés soient non enrobés au préalable, les pigments sont parfaitement dispersés dans la composition et le maquillage obtenu est homogène.

### Exemple 2

On prépare des compositions autobronzantes comprenant (en gramme) :

| | Composition D | Composition E | Composition F |
|---|---|---|---|
| A) polydiméthylsiloxane à 12-14 % dans le cyclopentasiloxane (Q2-1401) | 67,50 | 67,50 | 67,50 |
| B) TiO₂ + oxydes de fer (non enrobés) | 3,36 | 3,36 | 3,36 |
| C) . poudre de silicone réticulée dans un PDMS (KSG 16 de Shin Etsu) | 20,00 | 10,00 | 10,00 |
| . bentone gel VS-5 PC (Dubois) | -- | 10,00 | -- |
| . Unitwis (United Guardian) | -- | -- | -- |
| D) polyvinylidène | 1,00 | 1,00 | 1,00 |
| E) huiles de silicone | | | |
| . alkyl diméthicone X2-1731 (Dow) | -- | -- | 5,00 |
| . cyclopentadiméthylsiloxane | qsp 100 | qsp 100 | qsp 100 |

On obtient des crèmes gélifiées épaisses, d'application facile et agréable, donnant une coloration de la peau homogène, uniforme, d'aspect naturel.

### Exemple 3

On prépare des compositions autobronzantes siliconées résistantes à l'eau ayant les compositions suivantes (en g) :

| | Composition G | Composition H |
|---|---|---|
| A) . huile de silicone (AK 500 000 de Wacker) | 10,00 | -- |
| . diméthylpolysiloxane à 12-14 % dans cyclométhicone (Q2-1401 de Dow) | 6,64 | 12,30 |
| . polyphénylsiloxane à 15 % dans cyclopentadiméthylsiloxane (Silbione 71634 de Rhône-Poulenc) | -- | 40,00 |
| B) TiO₂ + oxydes de fer (non enrobés) | 3,36 | 7,7 |
| C) poudre de silicone réticulée | 20,00 | 10,00 |
| D) gélifiant | -- | 18,00 |
| E) billes de résine siliconées (Tospearl de Toshiba) | -- | 5,00 |
| F) cyclopentadiméthylsiloxane | qsp 100 | qsp 100 |

On obtient des compositions auto-bronzantes sous forme de gels, résistantes à l'eau et colorées.

### Exemple 4

On prépare un rouge à lèvres ayant la composition suivante, en g :

| | |
|---|---|
| . diméthylpolysiloxane à 12-14% dans cyclométhicone (Q2-1401) | 70,00 |
| . poudre de silicone réticulée dans PDMS (KSG 16 de Shin Etsu) | 10,00 |
| . cyclopentadiméthylsiloxane | 10,00 |
| . oxyde de titane enrobé silicone | 2,83 |
| . oxydes de fer enrobés silicone | 4,70 |
| . D&C red n°7 | 0,47 |
| . micatitane | 2,00 |

On obtient une crème gélifiée fluide de couleur rose-rouge à effet irisé, douce à l'application, présentant une bonne tenue.

### Exemple 5

On prépare un fluide bronzant filtrant ayant la composition suivante, en g :

| | | |
|---|---|---|
| . diméthylpolysiloxane à 12-14% dans cyclométhicone (Q2-1401) | | 70,00 |
| . filtres UV | Parsol MCX de BASF | 7,00 |
| | Hydroxy 4 méthoxy-benzophénone | 2,00 |
| | FINSOLV TN (C₁₂-C₁₅ alcoolbenzoate de Finetex | 9,00 |
| . huile de jojoba | | 1,00 |
| . acétate d'α-tocophérol | | 1,00 |
| . éthanol | | 4,70 |
| . parfum | | 0,30 |
| . poudre de silicone réticulée dans PDMS (KSG 16 de Shin Etsu) | | 10,00 |
| . TiO₂ + oxydes de fer (non enrobés) | | 1,70 |

On obtient un fluide bronzant, de couleur marron, lisse, brillant, d'application facile et restituant une coloration de la peau homogène, uniforme et d'aspect naturel.

## Revendications

1. Composition cosmétique anhydre résistante à l'eau comprenant
. 2% à 50 % en poids d'une gomme de silicone répondant à la formule : 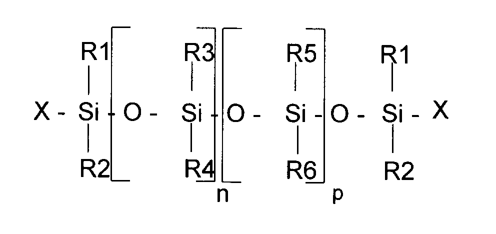 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant 1 à 6 atomes de carbone, ou un radical aryle,
X est un radical alkyle ayant 1 à 6 atomes de carbone, un radical hydroxyle ou un radical vinyle,
n et p étant choisis de manière à conférer à la gomme de silicone une viscosité supérieure à 100 000 mPa.s.,
. 10% à 90% en poids d'une huile de silicone,
. 0,5% à 15 % en poids de pigments et
. 0-30% en poids de charges.

2. Composition selon la revendication 1, dans laquelle la gomme de silicone est présente en une proportion comprise entre 4 et 15% en poids, de préférence entre 6 et 9% en poids, par rapport au poids total de la composition

3. Composition selon l'une des revendications précédentes, dans laquelle n et p ont, chacun, une valeur comprise entre 0 et 5000, de préférence entre 0 et 3000.

4. Composition selon l'une des revendications précédentes, dans laquelle la gomme de silicone est choisie dans le groupe constitué par les gommes pour lesquelles
. les substituants R1 à R6 et X représentent un groupement méthyle, p = 0 et n = 2700;
. les substituants R1 à R6 et X représentent un groupement méthyle, p = 0 et n = 2300;
. les substituants R1 à R6 représentent un groupement méthyle, le substituant X représente un groupement hydroxyle, p = 0 et n = 2700;
. les substituants R1, R2, R5, R6 et X représentent un groupement méthyle et les substituants R3 et R4 représentent un groupement aryle tel que le poids moléculaire de la gomme est de l'ordre de 600 000.

5. Composition selon l'une des revendications précédentes, dans laquelle l'huile de silicone est présente en une proportion comprise entre 30 et 70% en poids.

6. Composition selon l'une des revendications précédentes, dans laquelle l'huile de silicone est choisie dans le groupe constitué des cyclométhicones, des polydiméthylsiloxanes de viscosité inférieure à 100 mPa.s, et des alkyldiméthicones.

7. Composition selon l'une des revendications précédentes, dans laquelle les pigments sont présents en une proportion de 2 à 8% en poids par rapport au poids total de la composition.

8. Composition selon l'une des revendications précédentes, dans laquelle les pigments sont des pigments minéraux ou organiques, blancs ou colorés, enrobés ou non enrobés.

9. Composition selon l'une des revendications précédentes, dans laquelle les pigments sont choisis parmi le dioxyde de titane, l'oxyde de zinc, le dioxyde de zirconium, les oxydes de fer, le dioxyde de cérium, les pigments organiques dits laques de baryum, strontium, calcium, aluminium, et leurs mélanges.

10. Composition selon l'une des revendications précédentes se présentant sous la forme d'un produit de maquillage tel qu'un rouge à lèvres, un fard à joues, un fard à paupières ou un fond de teint, ou sous la forme d'un produit capillaire tel qu'un gel maquillant coiffant.

## Claims

1. Anhydrous, water-resistant cosmetic composition comprising
· 2% to 50% by weight of a silicone gum corresponding to the formula: 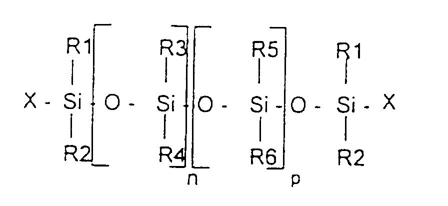 in which
R₁, R₂, R₅ and R₆ are, together or separately, an alkyl radical having 1 to 6 carbon atoms,
R₃ and R₄ are, together or separately, an alkyl radical having from 1 to 6 carbon atoms, or an aryl radical,
X is an alkyl radical having from 1 to 6 carbon atoms, a hydroxyl radical or a vinyl radical,
n and p being chosen so as to give the silicone gum a viscosity of greater than 100,000 mPa s,
· 10% to 90% by weight of a silicone oil,
· 0.5% to 15% by weight of pigments, and
· 0-30% by weight of fillers.

2. Composition according to Claim 1, in which the silicone gum is present in a proportion of between 4 and 15% by weight, preferably of between 6 and 9% by weight, relative to the total weight of the composition.

3. Composition according to one of the preceding claims, in which n and p each have a value of between 0 and 5000, preferably of between 0 and 3000.

4. Composition according to one of the preceding claims, in which the silicone gum is chosen from the group consisting of gums for which
· the substituents R1 to R6 and X represent a methyl group and p = 0 and n = 2700;
· the substituents R1 to R6 and X represent a methyl group and p = 0 and n = 2300;
· the substituents R1 to R6 represent a methyl group, the substituent X represents a hydroxyl group, p = 0 and n = 2700;
· the substituents R1, R2, R5, R6 and X represent a methyl group and the substituents R3 and R4 represent an aryl group such that the molecular weight of the gum is about 600,000.

5. Composition according to one of the preceding claims, in which the silicone oil is present in a proportion of between 30 and 70% by weight.

6. Composition according to one of the preceding claims, in which the silicone oil is chosen from the group consisting of cyclomethicones, polydimethylsiloxanes of viscosity less than 100 mPa s, and alkyldimethicones.

7. Composition according to one of the preceding claims, in which the pigments are present in a proportion of 2 to 8% by weight relative to the total weight of the composition.

8. Composition according to one of the preceding claims, in which the pigments are white or coloured, coated or non-coated inorganic or organic pigments.

9. Composition according to one of the preceding claims, in which the pigments are chosen from titanium dioxide, zinc oxide, zirconium dioxide, iron oxides, cerium dioxide, and organic pigments known as barium, strontium, calcium and aluminium lakes, and mixtures thereof.

10. Composition according to one of the preceding claims, which is in the form of a make-up product such as a lipstick, a blusher, an eyeshadow or a foundation, or in the form of a hair product such as a styling make-up gel.

## Patentansprüche

1. Wasserfreie wasserfeste kosmetische Zusammensetzung, die
· 2 bis 50 Gew.-% Silicongummi der Formel 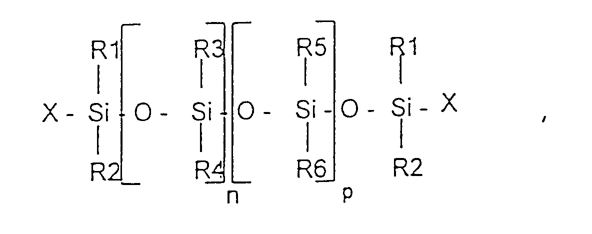 worin bedeuten:
R₁, R₂, R₅ und R₆, gleichzeitig oder unabhängig voneinander, eine Alkylgruppe mit 1 bis 6 Kohlenstoffatomen,
R₃ und R₄, gleichzeitig oder unabhängig voneinander, eine Alkylgruppe mit 1 bis 6 Kohlenstoffatomen oder eine Arylgruppe,
X eine Alkylgruppe mit 1 bis 6 Kohlenstoffatomen, eine Hydroxygruppe oder eine Vinylgruppe,
n und p Zahlen, die so ausgewählt sind, daß der Silicongummi eine Viskosität über 100 000 mPa·s aufweist,
· 10 bis 90 Gew.-% Siliconöl,
· 0,5 bis 15 Gew.-% Pigmente und
· 0 bis 30 Gew.-% Füllstoffe
enthält.

2. Zusammensetzung nach Anspruch 1, in der der Silicongummi in einem Mengenanteil im Bereich von 4 bis 15 Gew.-% und vorzugsweise von 6 bis 9 Gew.-%, bezogen auf das Gesamtgewicht der Zusammensetzung, vorliegt.

3. Zusammensetzung nach einem der vorhergehenden Ansprüche, in der n und p jeweils einen Wert im Bereich von 0 bis 5000 und vorzugsweise im Bereich von 0 bis 3000, aufweisen.

4. Zusammensetzung nach einem der vorhergehenden Ansprüche, in der der Silicongummi unter den Silicongummis ausgewählt ist, worin bedeuten:
· die Substituenten R1 bis R6 und X eine Methylgruppe, p = 0 und n = 2700,
· die Substituenten R1 bis R6 und X eine Methylgruppe, p = 0 und n = 2300,
· die Substituenten R1 bis R6 eine Methylgruppe, der Substituent X eine Hydroxygruppe, p = 0 und n = 2700,
· die Substituenten R1, R2, R5, R6 und X eine Methylgruppe und die Substituenten R3 und R4 eine Arylgruppe, so daß das Molekulargewicht des Gummis in der Größenordnung von 600 000 liegt.

5. Zusammensetzung nach einem der vorhergehenden Ansprüche, in der das Siliconöl in einem Mengenanteil im Bereich von 30 bis 70 Gew.-% vorliegt.

6. Zusammensetzung nach einem der vorhergehenden Ansprüche, in der das Siliconöl unter den Cyclometiconen, Polydimethylsiloxanen mit einer Viskosität unter 100 mPa·s und den Alkyldimeticonen ausgewählt ist.

7. Zusammensetzung nach einem der vorhergehenden Ansprüche, in der die Pigmente in einem Mengenanteil im Bereich von 2 bis 8 Gew.-%, bezogen auf das Gesamtgewicht der Zusammensetzung, vorliegen.

8. Zusammensetzung nach einem der vorhergehenden Ansprüche, in der die Pigmente anorganische oder organische, weiße oder gefärbte, umhüllte oder nicht umhüllte Pigmente sind.

9. Zusammensetzung nach einem der vorhergehenden Ansprüche, worin die Pigmente unter Titandioxid, Zinkoxid, Zirconiumdioxid, den Eisenoxiden, Cerdioxid, den organischen Pigmenten, die als Barium-, Strontium-, Calcium- und Aluminiumlacke bezeichnet werden, und deren Gemischen ausgewählt sind.

10. Zusammensetzung nach einem der vorhergehenden Ansprüche, die als Produkt zum Schminken, wie z.B. als Lippenstift, Wangenrouge, Lidschatten oder Make-up, oder als Produkt für das Haar, wie z.B. als Frisiergel oder Schminkgel vorliegt.
